(19) Europäisches Patentamt
European Patent Office
Office européen des brevets

(11) Publication number: **0 129 928**
**B1**

(12) # EUROPEAN PATENT SPECIFICATION

(45) Date of publication of patent specification: **02.09.87**

(21) Application number: **84200859.1**

(22) Date of filing: **14.06.84**

(51) Int. Cl.[4]: **C 07 D 413/04,**
C 07 D 417/04, A 01 N 43/80,
A 01 N 43/88

(54) **Oxazolyl-substituted diazaheterocycles.**

(30) Priority: **27.06.83 US 507816**
**27.06.83 US 507818**
**27.06.83 US 507874**
**27.06.83 US 507817**

(43) Date of publication of application:
**02.01.85 Bulletin 85/01**

(45) Publication of the grant of the patent:
**02.09.87 Bulletin 87/36**

(84) Designated Contracting States:
**AT BE CH DE FR GB IT LI LU NL SE**

(56) References cited:
**EP-A-0 002 881**
**EP-A-0 034 456**
**EP-A-0 044 185**
**CH-A- 447 187**
**US-A-4 354 030**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Pilgram, Kurt Hans Gerhard**
**2607 Warwick Lane**
**Modesto California 95350 (US)**

(74) Representative: **Hunter, Keith Roger Ian et al**
**4 York Road**
**London SE1 7NA (GB)**

**EP 0 129 928 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to certain oxazolyl-substituted diaza heterocycles which have been found to possess herbicidal activity, the preparation of such compounds, herbicidal compositions containing them, and to their use in combating undesired plant growth.

It is known from EP—A—34456, EP—A—44185 and US—A—4 354 030 that certain oxazole-substituted imidazolidinone compounds are herbicidally active. It is also shown in EP—A—44185 that an analogous imidazolinone compound shows some activity, but of a very low order, even at extremely high dosage rates.

It has now been discovered, surprisingly in view of the prior art, in particular EP—A—44185, that effective herbicidal action is exhibited by a new class of oxazolyl-substituted diaza heterocycles.

Accordingly, the present invention provides a compound of the formula

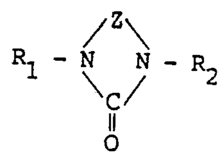

(I)

wherein Z is a bivalent group of the formula —CO—CO—, —CO—S—, —CO—CHOH—, or $—CH_2—X—CH_2$;

X is an oxygen atom or the group $N—R_3$ wherein $R_3$ is an alkyl group of from 1 to 3 carbon atoms, or a cyclopropyl or methylcyclopropyl group;

one of $R_1$ and $R_2$ is an oxazolyl group of formula IIA

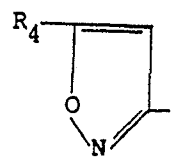

IIA

or, when Z is —CO—CO—, or —CO—CHOH—, may alternatively be the isomeric formula IIB

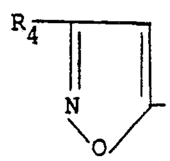

IIB

and the other is an alkyl, haloalkyl or alkoxy group of 1 to 3 carbon atoms, a cycloalkyl group of 3 to 6 carbon atoms or an alkenyl group of 3 to 5 carbon atoms.

and $R_4$ is a group containing 3 to 5 carbon atoms which is an alkyl, alkenyl, alkynyl, alkoxyalkyl, alkylthioalkyl, alkylsulphinylalkyl, alkylsulphonylalkyl, cycloalkyl, alkylcycloalkyl or haloalkyl group;

provided that when Z is —CO—CHOH then $R_2$ is not haloalkyl and $R_4$ is not alkoxyalkyl; and when Z is $—CH_2—X—CH_2—$ then $R_4$ is not haloalkyl.

In these compounds, each alkyl, alkoxy, alkenyl and alkynyl moiety suitably in either straight-chain or branched-chain in configuration. The term "halo" — i.e., halogen — designates one of chlorine, bromine and fluorine. Preferred alkylthioalkyl, alkylsulfinylalkyl and alkylsulfonylalkyl moieties are those of the formula:

$$\text{alkyl-S—(O)}_n\text{—}\underset{CH_3}{\overset{CH_3}{\underset{|}{\overset{|}{C}}}}\text{—}$$

wherein n is zero, one or two.

Preferred compounds are those wherein one of $R_1$ and $R_2$ represents an alkyl or alkoxy, especially methyl or methoxy, group; and those wherein $R_4$ represents an alkyl, alkynyl, alkoxyalkyl or alkylcycloalkyl, especially butyl, in particular tertiary butyl, ethynylpropyl, methoxybutyl or methylcyclopropyl, group. It is also preferred that the oxazolyl substituent has the isomeric form given in formula IIA, especially preferred being such compounds in which $R_4$ is tertiary butyl.

The invention also provides a process for the preparation of the compounds of the general formula I as defined above. For the compounds wherein Z represents —CO—CO—, —CO—S—, or —CO—CHOH, the compounds can be prepared by warming the appropriate urea of formula III

$$R_1\text{—NH—}\overset{O}{\overset{\|}{C}}\text{—NH—}R_2 \qquad III$$

with, respectively, oxalyl chloride, (chlorothio)formyl chloride or glyoxylic acid (conveniently as the hydrate), in the presence of an inert solvent.

In the first two cases the reaction may be represented by the equation:—

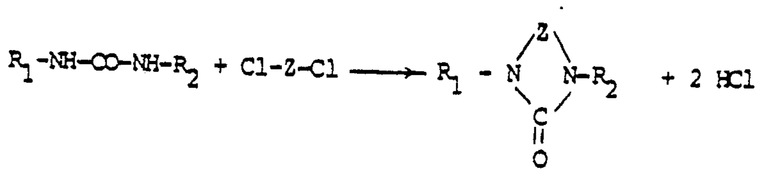

(Z being —COCO— or —CO—S—)

In the third case the reaction may be represented by the equation:—

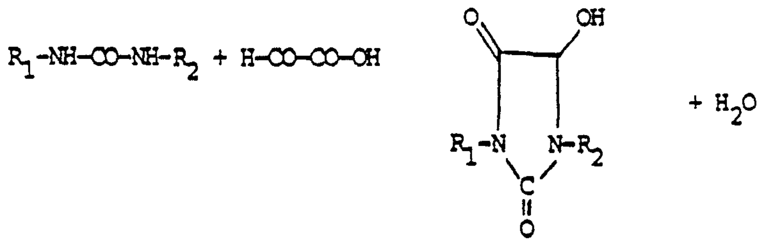

In some cases, because of the method of preparation, the urea reactant may be of the formula IVA or IVB

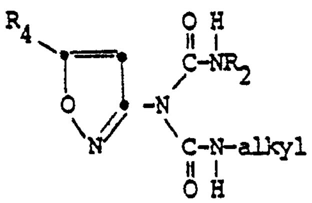

(IVA) or

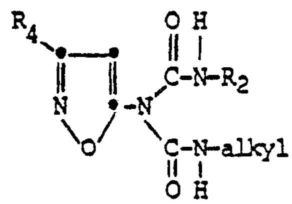

(IVB)

In some cases, the alkylaminocarbonyl moiety will be split off when the urea is heated in the presence of the chloride reactant. In other cases, it will be desirable, or necessary, to treat the urea of Formula IV with a base to split off the alkylaminocarbonyl moiety before the urea is treated with the chloride reactant. This procedure is illustrated in Example 6, hereinafter.

Treatment of the urea with oxalyl chloride or glyoxylic acid is conveniently conducted by adding the chloride or acid to a stirred solution of the urea in an inert solvent such as methylene chloride or toluene and refluxing the mixture for a sufficient time to enable the reaction to go to completion. The hydrogen chloride by-product in the first case may be removed as a gas as it is evolved, or absorbed by the incorporation of a hydrogen chloride acceptor such as a tertiary nitrogen base. The water by-product in the second case is preferably removed as a vapour as it evolves.

Treatment of the urea with (chlorothio)formyl chloride is conveniently conducted by adding the chloride to a stirred solution of the urea in a solvent at about room temperature in the presence of a tertiary nitrogen base as hydrogen chloride acceptor, chilling the resulting mixture to about 0°C, slowly adding the amine base thereto, then warming and stirring the mixture until the reaction is complete. In some cases, it may be found to be desirable to use a disulfide

$$(R—S—S—\overset{O}{C}—Cl,$$

wherein R is a hydrocarbon moiety) or ester

$$(Cl—S—\overset{O}{C}—O—R)$$

instead of (chlorothio)formyl chloride. In such cases, the intermediate product is cyclized by heat, in the case of the ester the cyclization being catalyzed by a base.

Those compounds wherein Z is $—CH_2OCH_2—$ can be prepared by treating the appropriate urea (Formula III) with paraformaldehyde in an inert solvent and in the presence of an acid, such as p-toluenesulfonic acid, as catalyst, the reaction proceeding according to the equation:

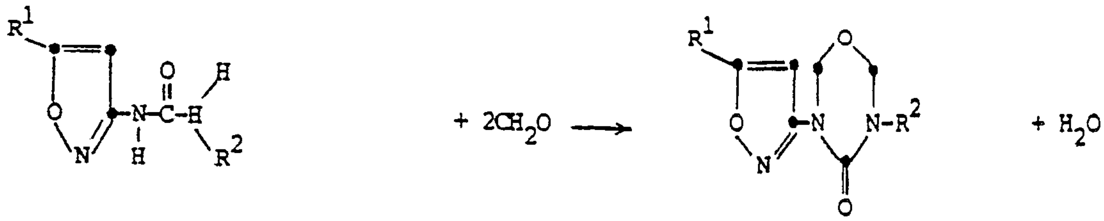

The treatment is conveniently conducted by heating a mixture of the urea, the paraformaldehyde, catalyst and solvent at a moderately elevated temperature — for example, 60—90°C. Aromatic hydrocarbons, such as toluene, are suitable solvents. If desired, a small amount — e.g., 10—30% of the amount of the amine, on a molar basis — may be included as a promoter.

Those compounds wherein Z is

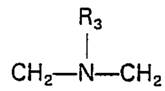

can be prepared by treating a solution of the appropriate urea

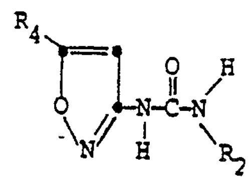

in an inert solvent with aqueous formaldehyde and treating the resulting mixture with the appropriate amine, $R_3$—$NH_2$. The reaction proceeds according to the equation:

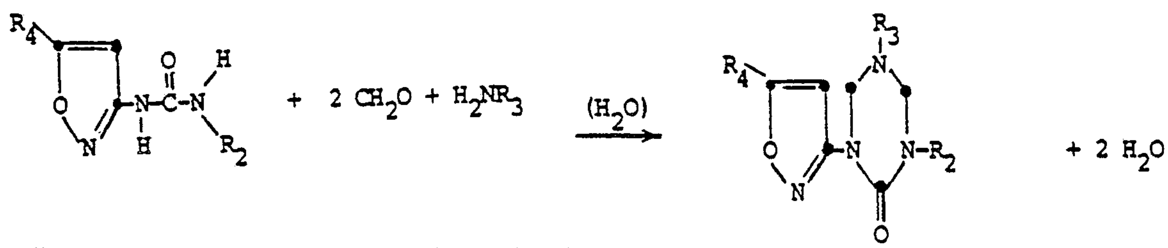


The treatment is conveniently conducted by slowly adding the formaldehyde solution to a stirred solution of the urea in the solvent, at room temperature, cooling if needed to maintain the temperature of the mixture below about 35°C, then slowly adding the amine, again cooling the mixture if needed to maintain its temperature below about 35°C. It may be found desirable to warm the mixture — for example, on a water bath, or even reflux it — after the amine has been added, to ensure completion of the reaction. Suitable solvents are aprotic liquids, such as dimethylformamide, dimethyl sulfoxide and sulfolane. Workup of the reaction mixture, isolation of the product and its purification are effected by conventional techniques, as illustrated in the examples, hereinafter.

Most of the oxazolylurea precursors (Formula III) are known compounds: U.S. Patent No. 4,062,861. Those wherein $R_4$ represents a moiety

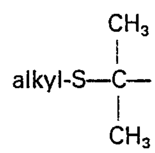

can be prepared by methods shown in that patent, the precursor amine being prepared from the nitrile, which in turn is prepared from methyl 2-methyl-2-methylthiopropionate, according to procedures described in Example 6, hereinafter. The ester can be prepared from methyl 2-bromo-2-methylpropionate (C. C. Price and E. C. Coyner, *J. Am. Chem. Soc.,* (1940) *62,* 1306—7) by the procedure shown in U.S. Patent 3,994,997 for the preparation of the ethyl ester.

These compounds of Formula I wherein $R_4$ is the moiety

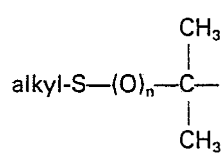

wherein n is one or two, can be prepared by conventional oxidation of the corresponding compounds of Formula I wherein n is zero. The oxidations can be effected by using such oxidizing reagents as hydrogen peroxide/acetic acid, peracids (for example meta-chloroperbenzoic acid), sodium metaperiodate, chromic acid/acetic acid, nitric acid, or potassium permanganate/acetic acid (or acetone), as described in "Methoden der Organischen Chemie", (Houben-Weyl), Schewfel-, Selen-, und Tellur-Verbindungen, vol. IX, pp. 207 & 208, Thieme Verlag, Stuttgart, Germany (1955).

The oxazolylamine precursor wherein $R_4$ = tertiary-butyl is the subject ot British Patent No. 2,014,992. The others can be prepared by the procedures described in that patent. Preparation of particular species of the precursor amines are shown in the examples, hereinafter.

The preparation, isolation and testing of typical individual species of the compounds of Formula I are described in the examples, following. The class of compounds is further illustrated and exemplified by the following further individual species, all of which are specifically contemplated in this invention. In the interest of brevity, in the identification of these species, the compounds are identified by reference to the meaning of the substituents in formula I, grouped according to the different meanings for Z.

(I) Z denotes —CO—CO—. This group is considered as being made up of two sub-classes:—

Subclass A, wherein the oxazolyl moiety is of Formula IIA, and Subclass B, wherein the oxazolyl moiety is of Formula IIB. The following are the species in question:

| Species No. | $R_2$ | $R_4$ |
|---|---|---|
| Subclass A: | | |
| 1 | methoxy | tertiary-butyl ($(CH_3)_3C—$) |
| 2 | cyclopropyl | tertiary-butyl |
| 3 | allyl | tertiary-butyl |
| 4 | propargyl ($CH{\equiv}C—CH_2—$) | tertiary-butyl |
| 5 | methyl | cyclopropyl |
| 6 | methyl | 1-methyl-1-methylthioethyl- ($CH_3—S—C(CH_3)_2—$) |
| 7 | methyl | 1-methyl-1-methylsulfenyl-ethyl ($CH_3—S(O)—C(CH_3)_2—$) |
| 8 | methyl | 1-methyl-1-methylsulfonyl-ethyl ($CH_3—S(O)_2—C(CH_3)_2—$) |

| Species No. | $R_2$ | $R_4$ |
|---|---|---|
| Subclass B: | | |
| 9 | methyl | tertiary-butyl |
| 10 | methyl | 1,1-dimethyl-2-propynyl ($CH{\equiv}C—C(CH_3)_2—$) |
| 11 | methoxy | 1,1-dimethyl-2-propynyl |
| 12 | methyl | cyclopropyl |
| 13 | methoxy | tertiary-butyl |
| 14 | allyl | tertary-butyl |
| 15 | propargyl | tertiary-butyl |

(II) Z denotes —CO—S—. This group is considered as being made up of two sub-classes: Subclass A,

wherein $R_1$ is the oxazolyl moiety, and Subclass B, wherein $R_2$ is the oxazolyl moiety. The following are the species in question:

| Species No. | $R_2$ | $R_4$ |
|---|---|---|
| Subclass A: | | |
| 16 | methoxy | tertiary-butyl ($(CH_3)_3C—$) |
| 17 | cyclopropyl | tertiary-butyl |
| 18 | isopropyl | tertiary-butyl |
| 19 | methyl | 1,1-dimethyl-2-propyn-1-yl ($HC{\equiv}C—C(CH_3)_2—$) |
| 20 | methyl | 1-methylcyclopropyl 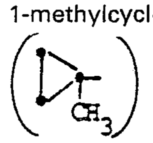 |
| 21 | methyl | 2-methoxy-1,1-di-methylethyl ($CH_3—O—CH_2—C(CH_3)_2—$) |
| 22 | methyl | 1-methyl-1-methylthioethyl ($CH_3—S—C(CH_3)_2—$ |
| 23 | methyl | 1-methyl-1-methylsulfenyl-ethyl ($CH_3—S(O)—C(CH_3)_2—$) |
| 24 | methyl | 1-methyl-1-methylsulfonyl-ethyl ($CH_3—S(O)_2—C(CH_3)_2—$) |
| Subclass B: | $R_1$ | $R_4$ |
| 25 | methyl | 1,1-dimethyl-2-propyn-1-yl |
| 26 | methoxy | tertiary-butyl |

(III) Z denotes —CO—CHOH—. This group is considered as being made up of two subclasses: Subclass A, wherein the oxazolyl moiety is $R_1$, and Subclass B, wherein the oxazolyl moiety is $R_2$. The following are the species in question:

| Species No. | $R_2$ | $R_4$ |
|---|---|---|
| Subclass A: | | |
| 27 | methoxy | tertiary-butyl ($(CH_3)_3C$—) |
| 28 | cyclopropyl | tertiary-butyl |
| 29 | allyl | tertiary-butyl |
| 30 | propargyl ($CH\equiv C$—$CH_2$—) | tertiary-butyl |
| 31 | methyl | cyclopropyl |
| 32 | methyl | 1,1-dimethyl-2-propynyl ($CH\equiv C$—$C(CH_3)_2$—) |
| 33 | cyclopropyl | 1,1-dimethyl-2-propynyl |
| 34 | methoxy | 1,1-dimethyl-2-propynyl |
| 35 | methyl | 1-methyl-1-methylthioethyl- ($CH_3$—S—$C(CH_3)_2$— |
| 36 | methyl | 1-methyl-1-methylsulfenyl-ethyl ($CH_3$—S(O)—$C(CH_3)_2$—) |
| 37 | methyl | 1-methyl-1-methylsulfonyl-ethyl ($CH_3$—$S(O)_2$—$C(CH_3)_2$—) |

| | $R_1$ | $R_4$ |
|---|---|---|
| Subclass B: | | |
| 38 | methyl | 1,1-dimethyl-2-propynyl |
| 39 | methoxy | 1,1-dimethyl-2-propynyl |
| 40 | methyl | cyclopropyl |
| 41 | methoxy | tertiary-butyl |
| 42 | allyl | tertiary-butyl |
| 43 | propargyl | tertiary-butyl |
| 44 | cyclopropyl | tertiary-butyl |
| 45 | methyl | 1-methylcyclopropyl |

(IV) Z denotes —$CH_2X$—$CH_2$

| Species No. | $R_3$ | $R_2$ | $R_1$ |
|---|---|---|---|
| X=>N—$R_3$ | | | |
| 46 | methyl | cyclopropyl | tertiary-butyl ($(CH_3)_3C—$) |
| 47 | methyl | allyl | tertiary-butyl |
| 48 | methyl | propargyl ($CH{\equiv}C—CH_2—$) | 1-methylcyclopropyl 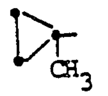 |
| 49 | methyl | methyl | cyclopropyl |
| 50 | methyl | methoxy | 1,1-dimethyl-2-propynyl) ($CH{\equiv}C—C(CH_3)_2—$) |
| 51 | propargyl | cyclopropyl | 1,1-dimethyl-2-propynyl |
| 52 | | methyl | 1-methyl-1-methylthioethyl ($CH_3—S—C(CH_3)_2—$) |
| 53 | | methyl | 1-methyl-1-methylsulfenyl-ethyl ($CH_3—S(O)C(CH_3)_2—$) |
| 54 | | methyl | 1-methyl-1-methylsulfonyl-ethyl ($CH_3—S(O)_2—C(CH_3)_2—$) |
| X = —O— | | | |
| 55 | | cyclopropyl | tertiary-butyl |
| 56 | | methyl | 1,1-dimethyl-2-propynyl |
| 57 | | methoxy | 1,1-dimethyl-2-propynyl |
| 58 | | cyclopropyl | 1,1-dimethyl-1-propynyl |

Compounds of Formula I have been found to affect adversely the growth of certain plants which are commonly considered as weeds, and also to have selectivity with respect to some crop plants — i.e., they control weeds at dosages at which they do not significantly harm the crop plants. Compounds of Formula I appear to be effective when applied preemergence or preplant incorporated (applied to the soil before the seeds have sprouted) or when applied postemergence (applied to the foliage of the growing plant). Some appear to be somewhat more effective when applied pre-emergence than when applied postemergence. Accordingly, the invention includes the use as a herbicide of a compound of formula I, and also a method of combating undesired plant growth at a locus by applying to the locus an effective amount of a compound of formula I. In the case where it is desired to control weeds in crop plantings, it is of course preferable to employ the lowest dosage that will control the weeds, for this will minimize any possible deleterious effect of the compound upon the crop plants. For application, the compound generally is applied most effectively by formulating it with a suitable inert carrier or surface-active agent, or both. The invention, therefore, also includes a herbicidal composition comprising a carrier or surface-active agent, or both, and as active ingredient at least one compound of Formula I, and a method of making such a composition which comprises bringing a compound of formula I into association with at least one carrier.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for

example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

The term "carrier" as used herein means an inert solid or fluid material, which may be inorganic or organic and of synthetic or natural origin, with which the active compound is mixed or formulated to facilitate its application to the plant, seed, soil or other object to be treated, or its storage, transport and/or handling. Any of the materials customarily employed in formulating pesticides, herbicides, or fungicides, are suitable.

Suitable solid carriers are natural and synthetic clays and silicates, for example, natural silicas such as diatomaceous earths; magnesium silicates, for example, talcs; magnesium aluminum silicates, for example, kaolinites, montmorillonites and micas; calcium carbonate; calcium sulfate; synthetic hydrated silicon oxides and synthetic calcium or aluminum silicates; elements such as, for example, carbon and sulfur; natural and synthetic resins such as, for exaple, coumarone resins, polyvinyl chloride and styrene polymers and copolymers; bitumen; waxes such as, for example, beeswax, paraffin wax, and chlorinated mineral waxes; solid fertilizers, for example, superphosphates; and ground, naturally-occurring, fibrous materials, such as ground corncobs.

Examples of suitable fluid carriers are water, alcohols such as, for example, isopropyl alcohol, glycols; ketones such as, for example, acetone, methyl ethyl ketone, methyl isobutyl ketone and cyclohexanone; ethers such as, for example, cellosolves; aromatic hydrocarbons such as, for example, kerosene, light mineral oils; chlorinated hydrocarbons such as, for example, carbon tetrachloride, perchloroethylene, trichloroethane, including liquefied, normally vaporous, gaseous compounds. Mixtures of different liquids are often suitable.

The surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3—10% w of a dispersing agent and, where necessary, 0—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—75% w active ingredient and 0—10% w of additives such as stabilisers, surfactants, slow release modifiers and binding agents. Emulsifiable concentrates usually contain, in addition to a solvent and, when necessary, co-solvent, 10—50% weight per volume active ingredient, 2—20% weight per volume emulsifiers and 0—20% weight per volume of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties, as are appropriate to the intended purpose.

Protection of a locus or area from undesirable plants is effected by applying a compound of Formula I, ordinarily in a composition of one of the aforementioned types, to soil in which the seeds of the unwanted plants are present, or to the foliage of the unwanted plants. The active compound, of course, is applied in an amount sufficient to exert the desired action. The amount of the compound of the invention to be used

in combating undesired plants will naturally depend on the condition of the plants, the degree of activity desired, the formulation used, the mode of application, the climate, the season of the year, and other variables. Recommendations as to precise amounts are, therefore, not possible. In general, however, application to the locus to be protected of from 0.1 to 10.0 kg per hectare of the compound of Formula I will be satisfactory.

The preparation, isolation and physical properties of typical individual species of the compounds of Formula I, in particular instances, are described in the following examples. In each case, the identity of the product, and each of any intermediate involved, was confirmed by appropriate chemical and spectral analyses.

## Example 1

1-Methyl-3-(5-(tertiary-butyl)-1,2-oxazol-3-yl)imidazolidinetrione (*1*)

A solution of 41.5 g of hydroxylamine hydrochloride in 136 ml of water was added drop-by-drop over a 10-minute period to a stirred, refluxing mixture of 56.1 g of pivaloylacetonitrile (British Patent 2,014,992), 700 ml of water and 525 ml of ethanol. The mixture was held under those conditions for 7 hours, the pH of the mixture being periodically adjusted to 6.5 by the addition of either hydrogen chloride or aqueous sodium hydroxide solution as required. Then the solvent ethanol was evaporated in a rotary evaporator, and the residual liquid was dried ($MgSO_4$), placed in 200 ml of hexane and chilled. Filtration and fractional crystallization of the solid gave 3-amino-5-(tertiary-butyl)-1,2-oxazole (*1A*), a yellow solid, m.p.: 107—109°C. A second fraction was 5-amino-3-(tertiary-butyl)-1,2-oxazole (*1B*), a solid, m.p.: 89—90°C.

*1A* was converted to 1-methyl-3-(5-(tertiary-butyl)-1,2-oxazol-3-yl)urea (*1C* by the method and procedures disclosed in U.S. Patent No. 4,062,861.

3.6 g of oxalyl chloride was added to a solution of 5.0 g of *1C* in 250 ml of toluene. The solution was refluxed for 15 minutes, then the solvent was evaporated under reduced pressure. The residue was crystallized from ether-hexane to give *1*, a colorless solid, m.p.: 127—128°C.

## Example 2

1-Methyl-3-(3-tertiary-butyl)-1,2-oxadiazol-5-yl)imidazolidinetrione (*2*)

A mixture of 2 g of *1B*, 3 g of methyl isocyanate and 10 ml of tetrahydrofuran containing 0.1 ml of triethylamine was stirred and refluxed for 22 hours. Then it was cooled to 20°C, filtered, and washed with cold ether. Recrystallization from ether gave 1-methyl-3-(3-(tertiary-butyl)-1,2-oxazol-5-yl)urea (*2A*), as a white solid, m.p.: 198—199°C.

*2* was prepared as a solid, m.p.: 176—179°C from *2A* and oxalyl chloride by the method described in Example 1 for converting *1C* to *1*.

## Example 3

1-Methyl-3-(5-(1-methylcyclopropyl)-1,2-oxazol-3-yl)-imidazolidinetrione (*3*)

A mixture of 61.1 g of acetonitrile and 108.3 g of methyl 1-methylcyclopropanecarboxylate was added drop-by-drop over a one-hour period to a stirred, refluxing mixture of 35.8 g of sodium hydride and 650 ml of tetrahydrofuran. The resulting mixture was stirred and refluxed for a further 18 hours, then most of the solvent was evaporated in a rotary evaporator. The residue was dissolved in 1 liter of water, and the solution was acidified with hydrochloric acid and extracted with ether. The extract was dried ($MgSO_4$) and concentrated. The residue, a liquid, was fractionally distilled to give (1-methylcyclopropylcarbonyl)-acetonitrile (*3A*) as a colorless oil which crystallized on standing, m.p.: 32—33°C.

*3A* was converted to 3-amino-5-(1-methylcyclopropyl)-1,2-oxazole (*3B*), a white solid m.p.: 82—84°C, and 5-amino-3-(1-methylcyclopropyl)-1,2-oxazole (*3C*), a white solid, m.p.: 106—109°C, by the method described for the preparation of *1A* and *1B*, Example 1.

A solution of 1.0 g of *3B* and 0.6 g of methyl isocyanate in 15 ml of ether containing one drop of triethylamine was held at 22°C for 18 hours. Then the mixture was cooled to −10°C and filtered, giving 1-methyl-3-(5-(1-methylcyclopropyl)-1,2-oxazol-3-yl)urea (*3D*), as a colorless solid, m.p.: 144—147°C.

*3D* was converted to *3*, as a colorless solid, m.p.: 124—126°C, by the method described in Example 1 for converting *1C* to *1*.

## Example 4

1-Methyl-3-(3-(1-methylcyclopropyl)-1,2-oxazol-5-yl)-imidazolidinetrione (*4*)

A solution of 1.0 g of *3C* and 0.6 g of methyl isocyanate in 15 ml of ether containing one drop of triethylamine was held at 22°C for 24 hours. No reaction took place. The mixture was placed in a sealed glass cylinder and heated for 24 hours on a steam bath. Then the solvent was evaported and the residue was chromatographed over silica gel using as eluent Solvent No. 3 (a 2:15:33 v:v:v mixture of tetrahydrofuran, ethyl acetate and hexane), to give 3-bis(methylaminocarbonylamino)-3-(1-methylcyclopropyl)-1,2-oxazole (*4A*), as a white solid m.p.: 125—127°C.

*4A* was converted to *4*, as an off-white solid, m.p.: 125—127°C, by the method described in Example 1 for converting *1C* to 1.

Example 5

1-methyl-3-(5-(1-methoxymethyl)-1-methylethyl)-1,2-oxazol-3-yl)imidazolidinetrione (*5*)

219 g of chloropivaloyl chloride was added drop-by-drop to a chilled (0—5°C), stirred solution of 141.4 g of triethylamine in 320 g of anhydrous methanol. The resulting mixture was warmed to room temperature in one hour, and most of the methanol was evaporated under reduced pressure. The residue was drowned in water and extracted with ether. The extract was dried ($MgSO_4$) and the solvent was evaporated. The residue was distilled to give methyl 3-chloro-2,2-dimethylpropionate (*5A*), b.p.: 40—50°C (14 Torr.).

A solution of 168.3 g of *5A* in 76 g of acetonitrile and 118 ml of tetrahydrofuran was added drop-by-drop over a one-hour period to a stirred, refluxing mixture of 42.2 g of sodium hydride and 766 ml of tetrahydrofuran. The resulting mixture was stirred and refluxed for 18 hours, then part of the solvent was evaporated. The residue was drowned in 1 liter of ice water, the resulting mixture was acidified with hydrochloric acid and extracted with ether. The extract was dried ($MgSO_4$), concentrated and distilled to give ((1-(methoxymethyl)-1-methylethyl)carbonyl)acetonitrile (*5B*), as a colorless liquid, b.p.: 86—90°C (0.2 Torr.).

A solution of 58.5 g of hydroxylamine hydrochloride in 88 ml of water was added drop-by-drop over a 10-minute period to a stirred, refluxing solution of 100.9 g of *5B* and 33.7 g of sodium hydroxide in 950 ml of water and 740 ml of ethanol. The mixture was stirred and refluxed for a further 18 hours, then the ethanol was evaporated on a rotary evaporator. The aqueous residue was cooled and filtered. The solid was chromatographed over silica gel, using Solvent No. 3 as eluent, to give 3-amino-5-(1-(methoxymethyl)-1-methylethyl)-1,2-oxazole (*5C*), as a cream-colored solid, m.p.: 61—64°C.

*5C* was converted to 1-methyl-3-(5-(1-methoxymethyl)-1-methylethyl)-1,2-oxazol-3-yl)urea (*5D*), as a solid, m.p.: 113—115°C, by the procedures described in Example 3 for converting *3B* to *3D*.

*5D* was converted to *5*, a solid, m.p.: 93—94°C, by the method described in Example 1 for converting *1C* to *1*.

Example 6

1-methyl-3-(5-(1,1-dimethyl-2-propyn-1-yl)-1,2-oxazol-3-yl)imidazolidinetrione (*6*)

The methyl ester of 2,2-dimethyl-3-butynoic acid (*6A*) was prepared from the acid (M. A. Schexnayder and P. S. Engel, Journal of the American Chemical Society, Vol. 97, pp 4825 et seq. (1975)) by conventional procedures.

A mixture of 109.8 g of *6A* and 56.2 g of acetonitrile was added drop-by-drop to a stirred refluxing mixture of 72.5 g of sodium hydride and 650 ml of tetrahydrofuran. The mixture was stirred and refluxed for 2 hours, then cooled to 20°C and stirred for 18 hours. Then the solvent was evaporated in a rotary evaporator, the residue was drowned in 500 ml of ether, and water was added cautiously. The resulting aqueous phase was separated, acidified with hydrochloric acid and extracted with ether. The extract was dried ($MgSO_4$) and the solvent was evaporated. The residue, a dark viscous syrup, was distilled to give ((1,1-dimethyl-2-propyn-1-yl)carbonyl)acetonitrile (*6B*), a yellow liquid, b.p.: 65—67°C (0.5 Torr.).

Over a 15-minute period, 21.0 g of *6B* was added to a solution of 9.1 g of sodium hydroxide in 240 ml of water and 200 ml of ethanol at room temperature. The pH of the mixture was adjusted to 6.4—6.6. The mixture was stirred, heated to 90°C and stirred at that temperature for 8 hours. The ethanol was evaporated under reduced pressure and the aqueous residue was extracted with ether. The extract was dried ($MgSO_4$) and concentrated. The resulting amber syrup was chromatographed over silica gel, using Solvent No. 3 as eluent, to give 3-amino-5-(1,1-dimethyl-2-propyn-1-yl)-1,2-oxazole (*6C*), as an off-white solid, m.p.: 98—100°C.

8.0 g of *6C* was treated with methyl isocyanate by the method described for treatment of *3C* in Example 4, to give 3-bis(methylaminocarbonylamino)-5-(1,1-dimethyl-2-propyn-1-yl)-1,2-oxazole (*6D*), as a light amber syrup.

A mixture of 5.4 g of *6D*, 1.2 g of sodium hydroxide, 10 ml of water and 50 ml of tetrahydrofuran was stirred and refluxed for 6 hours. Then it was concentrated under reduced pressure, the residue was diluted with 50 ml of water, acidified with dilute hydrochloric acid and extracted with methylene chloride. The extract was dried ($MgSO_4$), filtered and concentrated. The residue was recrystallized from ether-hexane to give 1-methyl-3-(5-(1,1-dimethyl-2-propyn-1-yl)-1,2-oxazol-3-yl)urea (*6E*), as a cream-colored solid, m.p.: 117—120°C.

*6E* was treated with oxalyl chloride, by the method described for treatment of *1C*, Example 1, to give 6, as a tan solid, m.p.: 99—101°C.

Example 7

4-Methyl-2-((5-tertiary-butyl)-1,2-oxazol-3-yl)-1,2,4-thiadiazolidine-3,5-dione (*7X*); 2-Methyl-4-((5-tertiary-butyl)-1,2-oxazol-3-yl)-1,2,4-thiadiazolidine-3,5-dione (*7Y*)

A solution of 41.5 g of hydroxylamine hydrochloride in 136 ml of water was added drop-by-drop over a 10-minute period to a stirred, refluxing mixture of 56.1 g of pivaloylacetonitrile (British Patent 2,014,992), 700 ml of water and 525 ml of ethanol. The mixture was held under those conditions for 7 hours, the pH of the mixture being periodically adjusted to 6.5 by the addition of either hydrogen chloride or aqueous sodium hydroxide solution, as required. Then the solvent ethanol was evaporated in a rotary evaporator,

the residual liquid was dried ($MgSO_4$), and placed in 200 ml of hexane and chilled. Filtration and fractional crystallization of the solid gave 3-amino-5-(tertiary-butyl)-1,2-oxazole (*7A*), a yellow solid, m.p.: 107—109°C. A second fraction was 5-amino-3-(tertiary-butyl)-1,2-oxazole (*7B*), a solid, m.p. 89—90°C.

*7A* was converted to 1-methyl-3-(5-(tertiary-butyl)-1,2-oxazol-3-yl)urea (*7C*) by the method and procedures disclosed in U.S. Patent No. 4,062,861.

40.8 g of (chlorothio)formyl chloride was added to a stirred solution of 50.0 g of *7C* in 800 ml of methylene chloride at room temperature. The resulting solution was cooled and held at 10—15°C while 80.6 g of ethyldiisopropylamine was added drop-by-drop in two hours. The resulting mixture was stirred for 16 hours at room temperature, then mixed with water and extracted with methylene chloride. The extract was dried ($MgSO_4$), the solvent was evaporated, and the residue was absorbed on silica gel and eluted with a 1:4:20 v:v:v mixture of tetrahydrofuran, ethyl acetate and hexane. The first fraction that was obtained consisted of the starting urea. Upon recrystallization from ether-hexane, the second fraction gave *7X*, an off-white solid, m.p.: 113—114°C. The third fraction was *7Y*, a light yellow solid, m.p.: 114—115°C. The melting point of a mixture of *7X* and *7Y* was depressed (92—95°C).

Example 8

4-Methyl-((5-(1,1-dimethyl-2-methoxyethyl)-1,2-oxazol-3-yl)-1,2,4-thiadiazolidine-3,5-dione (*8*)

219 g of chloropivaloyl chloride was added drop-by-drop to a chilled (0—5°C), stirred solution of 141.4 g of triethylamine in 320 g of anhydrous methanol. The resulting mixture was warmed to room temperature in one hour, and most of the methanol was evaporated under reduced pressure. The residue was mixed with water and extracted with ether. The extract was dried ($MgSO_4$), and the solvent was evaporated. The residue was distilled to give methyl 3-chloro-2,2-dimethylpropionate (*8A*), b.p.: 40—50°C (14 Torr.).

A solution of 168.3 g of *8A* in 76 g of acetonitrile and 118 ml of tetrahydrofuran was added drop-by-drop over a one-hour period to a stirred, refluxing mixture of 42.2 g of sodium hydride and 766 ml of tetrahydrofuran. The resulting mixture was stirred and refluxed for 18 hours, then part of the solvent was evaporated. The residue was added to 1 liter of ice water, and the resulting mixture was acidified with hydrochloric acid and extracted with ether. The extract was dried ($MgSO_4$) and distilled to give ((1-(methoxymethyl)-1-methylethyl)carbonyl)acetonitrile (*8B*), as a colorless liquid, b.p.: 86—90°C (0.2 Torr.).

A solution of 58.5 g of hydroxylamine hydrochloride in 88 ml of water was added drop-by-drop over a 10 minute period to a stirred, refluxing solution of 100.9 g of *8B* and 33.7 g of sodium hydroxide in 950 ml of water and 740 ml of ethanol. The mixture was stirred and refluxed for 18 hours, then the ethanol was evaporated in a rotary evaporator. The aqueous residue was cooled and filtered. The solid was chromatographed over silica gel, using a 2:15:33 v:v:v mixture of tetrahydrofuran, ethyl acetate and hexane as eluent, to give 3-amino-5-(1-(methoxymethyl)-1-methylethyl)-1,2-oxazole (*8C*), as a cream-colored solid, m.p.: 61—64°C.

A solution of 1.0 g of *8C* and 1.0 g of methyl isocyanate in 15 ml of anhydrous ether was held at room temperature for 3 days. Then the solvent was evaporated and the residue was crystallized from hexane to give 1-methyl-3-(5-(1,1-dimethyl-2-methylethyl)-1,2-oxazol-3-yl)urea (*8D*), as a colorless solid, m.p.: 113—115°C.

10.0 g of *8D* was treated with (chlorothio)formyl chloride and ethyldiisopropylamine by the procedure described in Example 7, to give 8, as a light yellow solid, m.p.: 65—67°C.

Example 9

1-Methyl-3-(5-(tertiary-butyl)-1,2-oxazol-3-yl)-5-hydroxyimidazolidine-2,4-dione (*9X*); 3-Methyl-1-(5-(tertiary-butyl)-1,2-oxazol-3-yl)-5-hydroxyimidazolidine-2,4-dione (*9Y*)

A solution of 41.5 g of hydroxylamine hydrochloride in 136 ml of water was added drop-by-drop over a 10-minute period to a stirred, refluxing mixture of 56.1 g of pivaloylacetonitrile (British Patent 2,014,992), 700 ml of water and 525 ml of ethanol. The mixture was held under those conditions for 7 hours, the pH of the mixture being periodically adjusted to 6.5 by the addition of either hydrogen chloride or aqueous sodium hydroxide solution, as required. Then the solvent ethanol was evaporated in a rotary evaporator, and the residual liquid was dried ($MgSO_4$), placed in 200 ml of hexane and chilled. Filtration and fractional crystallization of the solid gave 3-amino-5-(tertiary-butyl)-1,2-oxazole (*9A*), a yellow solid, m.p.: 107—109°C. A second fraction was 5-amino-3-(tertiarybutyl)-1,2-oxazole (*9B*), a solid m.p.: 89—90°C.

*9A* was converted to 1-methyl-3-(5-tertiary-butyl-1,2-oxazol-3-yl)urea (*9C*) by the method and procedures disclosed in U.S. Patent No. 4,062,861.

6.4 g of glyoxylic acid hydrate was added to a stirred solution of 10.0 g of *9C* in 300 ml of methylene chloride at room temperature. The solution was refluxed for one hour, then the solvent was evaporated under reduced pressure. The residue was chromatographed over silica gel, using a 1:4:20 v:v:v mixture of tetrahydrofuran, ethyl acetate and hexane as eluent. The first fraction was a solid, which on recrystallization from ether-hexane gave *9Y*, as a colorless solid, m.p.: 128—130°C. The second fraction was *9X*, a light yellow syrup, which on standing crystallized, m.p.: 122—123°C.

Example 10

1-Methyl-3-(5-(1-methylcyclopropyl)-1,2-oxazol-3-yl)-5-hydroxyimidazolidine-2,4-dione (*10X*); 3-Methyl-1-(5-(1-methylcyclopropyl)-1,2-oxazol-3-yl)-5-hydroxyimidazolidine-2,4-dione (*10Y*)

A mixture of 61.1 g of acetonitrile and 108.3 g of methyl 1-methylcyclopropanecarboxylate was added drop-by-drop over a one-hour period to a stirred, refluxing mixture of 35.8 g of sodium hydride and 650 ml of tetrahydrofuran. The resulting mixture was stirred and refluxed for a further 18 hours, then most of the solvent was evaporated in a rotary evaporator. The residue was dissolved in 1 liter of water, and the solution was acidified with hydrochloric acid and extracted with ether. The extract was dried ($MgSO_4$) and concentrated. The residue, a liquid, was fractionally distilled (69—71°C (4.5 Torr.) to give (1-methylcyclopropylcarbonyl)acetonitrile (*10A*) as a colorless oil which crystallized on standing, m.p.: 32—33°C.

*10A* was converted to 3-amino-5-(1-methylcyclopropyl)-1,2-oxazole (*10B*), a white solid m.p.: 82—84°C, and 5-amino-3-(1-methylcyclopropyl)-1,2-oxazole (*10C*), a white solid, m.p.: 106—109°C, by the method described for the preparation of *9A* and *9B*, Example 9.

A solution of 1.0 g of *10B* and 0.6 g of methyl isocyanate in 15 ml of ether containing one drop of triethylamine was held at 22°C for 18 hours. Then the mixture was cooled to −10°C and filtered, giving 1-methyl-3-(5-(1-methylcyclopropyl)-1,2-oxazol-3-yl)urea (*10D*), as a colorless solid, m.p.: 144—147°C.

4.6 g of glyoxylic acid hydrate was added to a stirred solution of 2.0 g of *10D* in 200 ml of xylene. The resulting solution was refluxed for 30 minutes, then the solvent was evaporated under reduced pressure. The residue was chromatographed over silica gel, using Solvent No. 3 (a 2:15:33 v:v:v mixture of tetrahydrofuran, ethyl acetate and hexane) as eluent. Workup and recrystallization from methanol gave *10X* as a first fraction, as a colorless solid, m.p. 160—163°C. The second fraction was *10Y*, as a colorless solid, m.p.: 146—149°C.

Example 11

1-methyl-3-(5-(1,1-dimethyl-2-propyn-1-yl)-1,2-oxazol-3-yl)-5-hydroxyimidazolidine-2,4-dione (*11X*; 3-Methyl-1-(5-(1,1-dimethyl-2-propyn-1-yl)-1,2-oxazol-3-yl)-5-hydroxyimidazolidine-2,4-dione (*11Y*)

The methyl ester of 2,2-dimethyl-3-butynoic acid (*11A*) was prepared from the acid (M. A. Schexnayder and P. S. Engel, Journal of the American Chemical Society, Vol. 97, pp 4825 et seq. (1975)) by conventional procedures.

A mixture of 109.8 g of *11A* and 56.2 g of acetonitrile was added drop-by-drop to a stirred, refluxing mixture of 72.5 g of sodium hydride and 650 ml of tetrahydrofuran. The mixture was stirred and refluxed for 2 hours, then cooled to 20°C and stirred for 18 hours. Then the solvent was evaporated in a rotary evaporator, the residue was drowned in 500 ml of ether, and water was added cautiously. The resulting aqueous phase was separated, acidified with hydrochloric acid and extracted with ether. The extract was dried ($MgSO_4$) and the solvent was evaporated. The residue a dark viscous syrup, was distilled to give ((1,1-dimethyl-2-propyn-1-yl)carbonyl)acetonitrile (*11B*), a yellow liquid, b.p.: 65—67°C (0.5 Torr.).

Over a 15-minute period, 21.0 g of *11B* was added to a solution of 9.1 g of sodium hydroxide in 240 ml of water and 200 ml of ethanol at room temperature. The pH of the mixture was adjusted to 6.4—6.6. The mixture was stirred, heated to 90°C and stirred at that temperature for 8 hours. The ethanol was evaporated under reduced pressure and the aqueous residue was extracted with ether. The extract was dried ($MgSO_4$) and concentrated. The resulting amber syrup was chromatographed over silica gel, using as eluent Solvent No. 3, to give 3-amino-5-(1,1-dimethyl-2-propyn-1-yl)-1,2-oxazole (*11C*), as an off-white solid, m.p.: 98—100°C.

8.0 g of *11C* was treated with methyl isocyanate by the method described for treatment of *10B* in Example 10, to give 3-bis(methylaminocarbonylamino)-5-(1,1-dimethyl-2-propyn-1-yl)-1,2-oxazole (*11D*), as a light amber group.

A mixture of 5.4 g of *11D*, 1.2 g of sodium hydroxide, 10 ml of water and 50 ml of tetrahydrofuran was stirred and refluxed for 6 hours. Then it was concentrated under reduced pressure, the residue was diluted with 50 ml of water, acidified with dilute hydrochloric acid and extracted with methylene chloride. The extract was dried ($MgSO_4$), filtered and concentrated. The residue was recrystallized from ether-hexane to give 1-methyl-3-(5-(1,1-dimethyl-2-propyn-1-yl)-1,2-oxazol-3-yl)urea (*11E*), as a cream colored solid, m.p.: 117—120°C.

A mixture of 5.7 g of *11E*, 250 ml of toluene and 7.0 g of glyoxylic acid hydrate was heated for two hours. The solvent was evaporated and the residue was chromatographed over silica gel, using Solvent No. 3 as eluent. Two fractions, each consisting of an amber syrup, were obtained. The first fraction was identified as *11Y*; the second fraction was identified as *11X*.

Example 12

3,5-Dimethyl-1-(5-(tertiary-butyl)-1,2-oxazol-3-yl)hexahydro-1,3,5-triazine-2-one (*12*)

A solution of 41.5 g of hydroxylamine hydrochloride in 136 ml of water was added drop-by-drop over a 10-minute period to a stirred, refluxing mixture of 56.1 g of pivaloylacetonitrile (British Patent 2,014,992), 700 ml of water and 525 ml of ethanol. The mixture was held under those conditions for 7 hours, the pH of the mixture being periodically adjusted to 6.5 by the addition of either hydrogen chloride or aqueous sodium hydroxide solution, as required. Then the solvent ethanol was evaporated in a rotary evaporator, and the residual liquid was dried ($MgSO_4$), placed in 200 ml of hexane and chilled. Filtration and fractional

crystallization of the solid gave 3-amino-5-(tertiary-butyl)-1,2-oxazole (*12A*), a yellow solid, m.p.: 107—109°C.

*12A* was converted to 1-methyl-3-(5-(tertiary-butyl)-1,2-oxazol-3-yl)urea (*12C*) by the method and procedures disclosed in U.S. Patent No. 4,062,861.

24 ml of a 36% aqueous formaldehyde solution was added drop-by-drop to a stirred solution of 15.0 g of *12C* in 75 ml of dimethylformamide. The resulting mixture was stirred for 30 minutes, then 12 ml of a 40% aqueous methylamine solution was added drop-by-drop, the temperature of the mixture being held below 35°C. The resulting solution was stirred at room temperature for 30 minutes, then on a hot water bath for 4 hours, then the solvent was evaporated under reduced pressure. The residue was a colorless syrup that crystallized upon standing. Recrystallization from hexane gave *12*, as a colorless solid, m.p.: 100—102°C.

Example 13

3,5-dimethyl-1-(5-(1-(methoxymethyl)-1-methylethyl-1,2-oxazol-3-yl)hexahydro-1,3,5-triazin-2-one (*13*)

219 g of chloropivaloyl chloride was added drop-by-drop to a chilled (0—5°C), stirred solution of 141.4 g of triethylamine in 320 g of anhydrous methanol. The resulting mixture was warmed to room temperature in one hour, and most of the methanol was evaporated under reduced pressure. The residue was drowned in water and extracted with ether. The extract was dried ($MgSO_4$) and the solvent was evaporated. The residue was distilled to give methyl 3-chloro-2,2-dimethylpropionate (*13A*), b.p.: 40—50°C (14 Torr.).

A solution of 168.3 g of *13A* in 76 g of acetonitrile and 118 ml of tetrahydrofuran was added drop-by-drop over a one-hour period to a stirred, refluxing mixture of 42.2 g of sodium hydride and 766 ml of tetrahydrofuran. The resulting mixture was stirred and refluxed for 18 hours, then part of the solvent was evaporated. The residue was drowned in 1 liter of ice water, and the resulting mixture was acidified with hydrochloric acid and extracted with ether. The extract was dried ($MgSO_4$), concentrated and distilled to give ((1-(methoxymethyl)-1-methylethyl)carbonyl)acetonitrile (*13B*), as a colorless liquid, b.p.: 89—90°C (0.2 Torr.).

A solution of 58.5 g of hydroxylamine hydrochloride in 88 ml of water was added drop-by-drop over a 10-minute period to a stirred, refluxing solution of 100.9 g of *13B* and 33.7 g of sodium hydroxide in 950 ml of water and 740 ml of ethanol. The mixture was stirred and refluxed for a further 18 hours, then the ethanol was evaporated in a rotary evaporator. The aqueous residue was cooled and filtered. The solid was chromatographed over silica gel, using as eluent a 2:15:33 v:v:v mixture of tetrahydrofuran, ethyl acetate and hexane, to give 3-amino-5-(1-(methoxymethyl)-1-methylethyl)-1,2-oxazole (*13C*), as a cream-colored solid, m.p.: 96—99°C.

*13C* was converted to 1-methyl-3-(5-(1-(methoxymethyl)-1-methylethyl)-1,2-oxazol-3-yl)urea (*13D*), as a solid, m.p.: 113—115°C, by the procedures described in U.S. Patent No. 4,062,861.

16 ml of a 36% aqueous formaldehyde solution was added drop-wise to a stirred solution of 4.1 g of *13D* in 75 ml of dimethylformamide. The temperature of the mixture rose from 22°C to 27°C. The mixture was stirred was for 30 minutes, then 8 ml of a 40% solution of methylamine in water was added, drop-by-drop. The temperature of the mixture rose from 27°C to 36°C. The mixture was refluxed for 3 hours, then the solvent was evaporated under reduced pressure. The residue was mixed with cold water, and the resulting mixture was extracted with ether. The solvent was evaporated from the extract, and the residue was crystallized when hexane was added, to give *13*, as a white solid, m.p.: 49—50°C.

Example 14

3,5-Dimethyl-1-(5-(1,1-dimethyl-2-propynyl)-1,2-oxazol-3-yl)-hexahydro-1,3,5-triazin-2-one (*14*)

The methyl ester of 2,2-dimethyl-3-butynoic acid (*14A*) was prepared from the acid (M. A. Schexnayder and P. S. Engel, Journal of the American Chemical Society, Vol. 97, pp 4825 et seq. (1975)) by conventional procedures.

A mixture of 109.8 g of *14A* and 56.2 g of acetonitrile was added drop-by-drop to a stirred, refluxing mixture of 72.5 g of sodium hydride and 650 ml of tetrahydrofuran. The mixture was stirred and refluxed for 2 hours, then cooled to 20°C and stirred for 18 hours. Then the solvent was evaporated in a rotary evaporator, the residue was drowned in 500 ml of ether, and water was added cautiously. The resulting aqueous phase was separated, acidified with hydrochloric acid and extracted with ether. The extract was dried ($MgSO_4$) and the solvent was evaporated. The residue, a dark viscous syrup, was distilled to give ((1,1-dimethyl-2-propyn-1-yl)carbonyl)acetonitrile (*14B*), a yellow liquid, b.p.: 65—67°C (0.5 Torr.).

Over a 15-minute period, 21.0 g of *14B* was added to a solution of 9.1 g of sodium hydroxide in 240 ml of water and 200 ml of ethanol at room temperature. The pH of the mixture were adjusted to 6.4—6.6. The mixture was stirred, heated to 90°C and stirred at that temperature for 8 hours. The ethanol was evaporated under reduced pressure and the aqueous residue was extracted with ether. The extract was dried ($MgSO_4$) and concentrated. The resulting amber syrup was chromatographed over silica gel, using Solvent No. 3 as eluent, to give 3-amino-5-(1,1-dimethyl-2-propyn-1-yl)-1,2-oxazole (*14C*), as an off-white solid, m.p.: 98—100°C.

A solution of 7.5 g *14C* and 5 ml of methyl isocyanate in 50 ml of tetrahydrofuran was heated at 50—60°C for two days. The solvent was evaporated under reduced pressure and the residue was

chromatographed over silica gel, using as eluent a 1:4:20 v:v:v mixture of tetrahydrofuran, ethyl acetate and hexane, to give 1-methyl-3-(5-(1,1-dimethyl-2-propyn-1-yl)-1,2-oxazol-3-yl)urea (*14D*), as a white crystalline solid, m.p.: 99—100°C.

3 ml of a 36% aqueous formaldehyde solution was added drop-by-drop to a stirred solution of 1.0 g of *14D* in 25 ml of dimethylformamide, at room temperature. After 30 minutes of stirring, 1.5 ml of 40% aqueous methylamine solution was added drop-by-drop, to the mixture. The resulting mixture was stirred for 30 minutes at room temperature and then on a water bath for one hour, then was held at room temperature for 18 hours. The solvents were evaporated under reduced pressure in a rotary evaporator. The residue was chromatographed over silica gel, using as eluent a 2:15:13 v:v:v mixture of tetrahydrofuran, ethyl acetate and hexane, to give *14*, as a colorless solid, m.p.: 85—87°C, upon recrystallization from ether.

Example 15

5-methyl-3-methoxy-1-(5-(tertiary-butyl)-1,2-oxazol-3-yl)hexahydro-1,3,5-triazin-2-one (*15*)

99 g of phosgene was bubbled into 500 ml of ethyl acetate, then a solution of 71.6 g of *12A* in 400 ml of ethyl acetate was added drop-by-drop to the stirred mixture. The mixture was stirred at reflux temperature for five hours, left standing overnight at room temperature, and filtered. The solid was dried at room temperature for two days to give dimeric 3-isocyanato-5-(tertiary-butyl)-1,2-oxazole (*15A*), m.p.: 165—167°C.

8.5 g of methoxyamine was added very slowly to a stirred solution of 15 g of *15A* in 600 ml of tetrahydrofuran. The resulting mixture was filtered and the solid was triturated with ether to give 1-methoxy-3-(5-(tertiary-butyl)-1,2-oxazol-3-yl)urea (*15B*), m.p.: 145—148°C.

By the procedure described in the last paragraph of Example 12, *15B* was converted to *15*, which was isolated as a solid, m.p.: 90—92°C, by chromatographing the crude reaction product over silica gel, using a 1:1:2 v:v:v mixture of tetrahydrofuran, ethyl acetate and hexane as eluent, *15* being the first fraction collected.

Example 16

3-Methyl-5-(5-(tertiary-butyl)-1,2-oxazol-3-yl)-2,6-dihydro-1,3,5-oxadiazin-4-one (*16*)

A mixture of 5.0 g of *12C*, 0.75 g of paraformaldehyde, 1.3 g of para-toluenesulfonic acid, 0.51 g of dimethylformamide and 150 ml of toluene was stirred at 80°C for 15 minutes. The resulting mixture was poured into 200 ml of water and phase-separated. The toluene phase was dried ($MgSO_4$), filtered and concentrated in a rotary evaporator. The residue was triturated with hexane, and the liquid was chromatographed over silica gel, using a 20:4:1 v:v:v mixture of hexane, ethyl acetate and tetrahydrofuran as eluent. Workup gave *16*, as a colorless syrupy liquid.

Example 17

*Herbicidal Activity*

In the following trials, the species of plants that were tested were:

Barnyardgrass (watergrass) — *Enchinochloa crus-galli*
Large crabgrass — *Digitaria sanguinalis*
Downy brome — *Bromus tectorum*
Yellow foxtail — *Setaria lutescens*
Redroot pigweed — *Amaranthus retroflexus*
Sicklepod — *Cassia obtusifolia*
Velvetleaf — *Abutilon theophrasti*
Garden cress — *Lepidium sativum*
Johnsongrass — *Sorghum halepense*

Test Procedures

The preemergence (soil) herbicidal activity of the compounds was evaluated by planting seeds of barnyardgrass, garden cress, downy brome, velvetleaf, yellow foxtail, and sicklepod in test tubes, nominally measuring 25 × 200 millimeters, filled about three-quarters full of untreated soil, in each case covered on top with about 2.5 cubic centimeters of soil treated with a certain amount of the test compound. The treated soil applied to the tubes containing the barnyardgrass and cress seeds contained one milligram of the test compound per tube, and contained 0.1 milligram of the test compound per each tube containing the seeds of the other plants. The dosages were approximately 20 and 2.0 kg. of test compound per hectare, respectively. The seeds were planted on top of the treated soil and covered with about 1.5 cubic centimeters of untreated soil. The planted soil was held under controlled conditions of temperature, moisture, and light for 9 to 10 days. The amounts of germination and growth in each tube were evaluated on a 0 to 9 scale, the numeric ratings having the following meanings:

| Rating | Meaning |
|---|---|
| 9 | No living tissue |
| 8 | Plant severely damaged and expected to die |
| 7 | Plant badly damaged, but expected to live |
| 6 | Moderate damage, but complete recovery expected |
| 5 | Intermediate damage (probably unacceptable for crop plants) |
| 3—4 | Observable damage |
| 1—2 | Plant slightly affected, possibly by the chemical, possibly due to biological variability |
| 0 | No visible effect |

The postemergence (foliar) herbicidal activity of compounds of the invention was evaluation by spraying 10-day-old large downy brome plants in some cases, 6-day-old Johnsongrass plants in other cases, 9-day-old velvetleaf plants, 9-day-old yellow foxtail plants and 9-day-old sicklepod plants to runoff with a liquid formulation of the test compound. The crabgrass and pigweed plants were sprayed with 2.4 milliliters of a 0.25% solution (about ten kg. of the test compound per hectare), and other plants were sprayed with 2.4 milliliters of a 0.025% solution (about one kg. of the test compound per hectare). The sprayed plants were held under controlled conditions of temperature, moisture and light for 7 to 8 days, and the effect of the test compound was then evaluated visually, the results being rated on the 0 to 9 scale described above.

Results of the preemergence and postemergence herbicidal activity tests are set forth in Table I.

TABLE I
Herbicidal Activity

| Compound | Preemergence | | | | | | Postemergence | | | | | |
|---|---|---|---|---|---|---|---|---|---|---|---|---|
| | Barnyard-grass | Garden Cress | Downy Brome | Velvet-leaf | Yellow Foxtail | Sickle-pod | Crab-grass | Pig-weed | Johnson-grass | Velvet leaf | Yellow Foxtail | Sickle-pod |
| *1* | 9 | 9 | 7 | 9 | 8 | 9 | 9 | 6 | 3 | 9 | 5 | 9 |
| *2* | 7 | 8 | 0 | 3 | 0 | 2 | 3 | 5 | 1 | 2 | 0 | 3 |
| *3* | 9 | 9 | 0 | 7 | 6 | 7 | 6 | 9 | 2 | 6 | 2 | 6 |
| *4* | 8 | 9 | 2 | 9 | 6 | 8 | 5 | 9 | 2 | 4 | 2 | 4 |
| *5* | 9 | 9 | 4 | 9 | 6 | 8 | 9 | 9 | 0 | 6 | 2 | 4 |
| *6* | 9 | 9 | 6 | 8 | 6 | 8 | 6 | 9 | 0 | 6 | 0 | 6 |
| *7X* | 9 | 9 | 9 | 9 | 9 | 9 | 9 | 9 | 2 | 9 | 9 | 9 |
| *7Y* | 9 | 9 | 8 | 8 | 8 | 8 | 9 | 9 | 5 | 6 | 2 | 9 |
| *8* | 8 | 9 | 3 | 8 | 5 | 8 | 4 | 8 | 2 | 3 | 2 | 3 |
| *9X* | 9 | 9 | 6 | 9 | 8 | 9 | 9 | 9 | 2 | 8 | 4 | 9 |
| *9Y* | 9 | 9 | 3 | 9 | 4 | 9 | 8 | 5 | 3 | 7 | 0 | 7 |
| *10X* | 9 | 9 | 2 | 9 | — | 9 | 6 | 9 | 1 | 4 | 3 | 6 |
| *10Y* | 8 | 9 | 3 | 8 | 3 | 8 | 7 | 9 | 2 | 4 | 0 | 5 |
| *11X* | 8 | 9 | 7 | 9 | 7 | 9 | 4 | 8 | 0 | 7 | 2 | 7 |

TABLE I (continued)
Herbicidal Activity

| Compound | Preemergence | | | | | | Postemergence | | | | | |
|---|---|---|---|---|---|---|---|---|---|---|---|---|
| | Barnyard-grass | Garden Cress | Downy Brome | Velvet-leaf | Yellow Foxtail | Sickle-pod | Crab-grass | Pig-weed | Johnson-grass | Velvet leaf | Yellow Foxtail | Sickle-pod |
| *11Y* | 9 | 9 | 6 | 5 | 5 | 7 | 2 | 7 | 0 | 5 | 3 | 3 |
| *12* | 9 | 9 | 6 | 9 | 8 | 9 | 9 | 9 | 2 | 8 | 4 | 8 |
| *13* | 9 | 9 | 3 | 8 | 6 | 8 | — | 5 | 0 | 3 | 2 | 4 |
| *14* | 8 | 9 | 6 | 9 | 8 | 9 | 6 | 8 | 2 | 7 | 5 | 7 |
| *15* | 8 | 7 | 2 | 6 | 5 | 6 | 2 | 6 | 0 | 4 | 0 | 5 |
| *16* | 9 | 9 | 8 | 9 | 9 | 9 | 9 | 9 | 5 | 6 | 4 | 7 |

Example 18

*Herbicidal Selectivity*

In the following examples, the species of plants that were tested were:

Barnyardgrass
Downy brome
Johnsongrass
Wild oats — *Avena fatua*
Yellow foxtail
Goose grass — *Eleusine indica* L.
Yellow nutsedge — *Cyperus esculentus* L.
Cocklebur — *Xanthum pennsylvanicum*
Morning glory — *Ipomoea purpurea* L. (Roth)
Wild mustard — *Brassica kaber*
Redroot pigweed
Sicklepod
Velvetleaf
Corn — *Zea mays*
Cotton — *Gossypium hirsutum*
Rice — *Oryza sativa*
Grain sorghum — *Sorghum vulgare*
Soybeans — *Beta vulgaris*
Wheat — *Triticum aestivum*

Test Procedures

The preemergence activity of compounds of Formula I was further determined with respect to certain species of crop plants and common species of weeds, by spraying a formulation of the test compounds on soil in small pots in which seeds of the plants had been sown. The results of the tests were evaluated on the basis of the 0—9 scale described with respect to the earlier tests. The results of the tests are reported in Table II.

TABLE II

| | Rating of effect at indicated dosage (kg/ha) and timing | | | | | | | | | | |
|---|---|---|---|---|---|---|---|---|---|---|---|
| | Compound 1 | | Compound 3 | | | | | Compound 7X | | Compound 7Y | |
| | Pre-emergence | | Preemergence | | | Postemergence | | Preemergence | | Preemergence | |
| Plant Species | 0.25 | 0.0625 | 2.0 | 1.0 | 0.25 | 2.0 | 1.0 | 0.25 | 0.0625 | 0.25 | 0.0625 |
| Corn | 3 | 0 | 4 | 2 | 0 | 2 | 2 | 2 | 0 | 2 | 0 |
| Cotton | 0 | 2 | 5 | 0 | 0 | 9 | 8 | 0 | 0 | 3 | 0 |
| Rice | 9 | 8 | 9 | 8 | 5 | 2 | 2 | 9 | 7 | 8 | 0 |
| Grain Sorghum | 9 | 3 | 7 | 2 | 0 | 3 | 3 | 7 | 3 | 7 | 0 |
| Soybean | 9 | 3 | 9 | 9 | 0 | 9 | 9 | 9 | 2 | 8 | 3 |
| Sugar Beet | 9 | 9 | 9 | 9 | 0 | 6 | 6 | 9 | 9 | 9 | 9 |
| Wheat | 9 | 9 | 9 | 5 | 0 | 2 | 0 | 9 | 6 | 9 | 8 |
| Barnyard Grass | 9 | 6 | 9 | 9 | 0 | 1 | 0 | 8 | 6 | 8 | 7 |
| Downy Brome | 3 | 3 | — | — | — | 1 | 0 | 5 | 4 | 0 | 0 |
| Johnsongrass | 7 | 2 | 9 | 7 | 5 | 0 | 0 | 8 | 0 | 9 | 6 |
| Wild Oats | 9 | 6 | 9 | 9 | 5 | 0 | 0 | 9 | 5 | 9 | 9 |
| Yellow Foxtail | 7 | 5 | 8 | 8 | 4 | 7 | 6 | 7 | 0 | — | 6 |
| Yellow Nutsedge | 3 | 3 | 4 | 0 | — | 2 | 0 | — | — | — | 0 |

TABLE II (continued)

| Plant Species | Rating of effect at indicated dosage (kg/ha) and timing | | | | | | | | | | |
|---|---|---|---|---|---|---|---|---|---|---|---|
| | Compound 1 | | Compound 3 | | | | | Compound 7X | | Compound 7Y | |
| | Pre-emergence | | Preemergence | | | Postemergence | | Preemergence | | Preemergence | |
| | 0.25 | 0.0625 | 2.0 | 1.0 | 0.25 | 2.0 | 1.0 | 0.25 | 0.0625 | 0.25 | 0.0625 |
| Goose Grass | 9 | 3 | 9 | 9 | 0 | 0 | 0 | 9 | 0 | 9 | 9 |
| Cocklebur | 9 | 9 | — | 9 | — | 9 | 8 | 9 | 9 | 7 | 9 |
| Morning Glory | 9 | 9 | 9 | 7 | 4 | 7 | 7 | 9 | 9 | 9 | 0 |
| Mustard | 9 | 9 | — | — | — | 9 | 9 | 9 | 9 | 9 | 9 |
| Pigweed | 9 | 9 | 9 | 9 | 9 | 9 | 9 | 9 | 9 | 9 | 9 |
| Sicklepod | 9 | 9 | 9 | 9 | 0 | 9 | 9 | 9 | 3 | 9 | 9 |
| Velvetleaf | 9 | 9 | 9 | 9 | 0 | 9 | 9 | 9 | 4 | 9 | 9 |

TABLE II (continued)

| Plant Species | Rating of effect at indicated dosage (kg/ha) and timing | | | | | | | | | |
|---|---|---|---|---|---|---|---|---|---|---|
| | Compound 9X | | | | Compound 9Y | | | | Compound 12 | |
| | Pre-emergence | | Postemergence | | Preemergence | | Postemergence | | Preemergence | |
| | 0.25 | 0.0625 | 1.0 | 0.25 | 0.25 | 0.0625 | 1.0 | 0.25 | 0.25 | 0.0625 |
| Corn | 2 | 0 | 5 | 3 | 0 | 0 | 3 | 3 | 0 | 0 |
| Cotton | 2 | 2 | 9 | 5 | 4 | 0 | 7 | 6 | 0 | 0 |
| Rice | 9 | 6 | 2 | 0 | 6 | 4 | 3 | 2 | 9 | 5 |
| Grain Sorghum | 9 | 4 | 3 | 0 | 3 | 0 | 3 | 2 | 6 | 0 |
| Soybean | 9 | 5 | 7 | 6 | 3 | 2 | 7 | 6 | 8 | 2 |
| Sugar Beet | 9 | 9 | 9 | 7 | 9 | 9 | 9 | 6 | 9 | 9 |
| Wheat | 9 | 7 | 4 | 0 | 9 | 4 | 2 | 1 | 9 | 7 |
| Barnyard Grass | 7 | 6 | 3 | 0 | 4 | 0 | 2 | 0 | 8 | 0 |
| Downy Brome | 6 | 0 | 4 | 0 | 0 | 0 | — | — | 3 | 0 |
| Johnsongrass | 7 | 5 | 3 | 0 | 2 | 0 | 4 | 2 | 7 | 0 |

TABLE II (continued)

| Plant Species | Rating of effect at indicated dosage (kg/ha) and timing | | | | | | | | | |
|---|---|---|---|---|---|---|---|---|---|---|
| | Compound 9X | | | | Compound 9Y | | | | Compound 12 | |
| | Pre-emergence | | Postemergence | | Preemergence | | Postemergence | | Preemergence | |
| | 0.25 | 0.0625 | 1.0 | 0.25 | 0.25 | 0.0625 | 1.0 | 0.25 | 0.25 | 0.0625 |
| Wild Oats | 9 | 8 | 6 | 0 | 4 | 0 | 5 | 2 | 9 | 0 |
| Yellow Foxtail | 6 | 3 | — | — | 0 | 0 | 2 | 0 | 9 | 0 |
| Goose Grass | 9 | 2 | 7 | 2 | 0 | 0 | 7 | 3 | 8 | 0 |
| Yellow Nutsedge | 0 | 0 | 3 | 0 | 3 | 0 | 3 | 1 | 0 | 0 |
| Cocklebur | 9 | 8 | 9 | 7 | 9 | 0 | — | — | 9 | 0 |
| Morning Glory | 9 | 9 | 8 | 6 | 9 | 9 | 7 | 4 | 9 | 7 |
| Mustard | 9 | 9 | 9 | 7 | 9 | 7 | 6 | 3 | 9 | 5 |
| Pigweed | 9 | 9 | 9 | 8 | 9 | 7 | 7 | 6 | 9 | 5 |
| Sicklepod | 9 | 9 | 9 | 7 | 9 | 8 | 4 | 2 | 9 | 0 |
| Velvetleaf | 9 | 8 | 8 | 6 | 9 | 8 | 7 | 6 | 9 | 3 |
| Nightshade | — | — | 9 | 8 | — | — | 9 | 8 | | |

**Claims for the Contracting States: BE CH DE FR GB IT LI LU NL SE**

1. A compound of the formula:

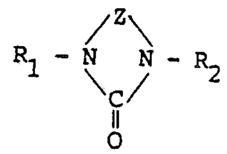

(I)

wherein

Z is a bivalent group of the formula —CO—CO—, —CO—S—, —CO—CHOH—, or $—CH_2—X—CH_2$;

X is an oxygen atom or the group $N—R_3$ wherein $R_3$ is an alkyl group of from 1 to 3 carbon atoms, or a cyclopropyl or methylcyclopropyl group;

one of $R_1$ and $R_2$ is an oxazolyl group of formula IIA

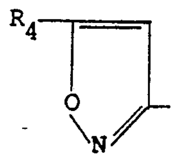

IIA

or, when Z is —CO—CO—, or —CO—CHOH—, may alternatively be the isomeric formula IIB

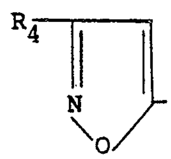

IIB

and the other is an alkyl, haloalkyl or alkoxy group of 1 to 3 carbon atoms, a cycloalkyl group of 3 to 6 carbon atoms or an alkenyl group of 3 to 5 carbon atoms; and

$R_4$ is a group containing 3 to 5 carbon atoms which is an alkyl, alkenyl, alkynyl, alkoxyalkyl, alkylthioalkyl, alkylsulphinylalkyl, alkylsulphonylalkyl, cycloalkyl, alkylcycloalkyl or haloalkyl group;

provided that when Z is —CO—CHOH then $R_2$ is not haloalkyl and $R_4$ is not alkoxyalkyl; and when Z is $—CH_2—X—CH_2—$ then $R_4$ is not haloalkyl.

2. A compound as claimed in claim 1 wherein one of $R_1$ and $R_2$ represents an alkyl or alkoxy group.

3. A compound as claimed in claim 1 or 2 wherein $R_4$ represents an alkyl, alkynyl, alkoxyalkyl or alkylcycloalkyl group.

4. A compound as claimed in claim 2 and 3 wherein one of $R_1$ and $R_2$ represents methyl or methoxy and $R_4$ represents a butyl, ethynylpropyl, methoxybutyl or methylcyclopropyl group.

5. A compound as claimed in claim 4 wherein $R_4$ is tertiary butyl.

6. A compound as claimed in any one of the preceding claims wherein the oxazolyl substituent has the isomeric form given in formula IIA.

7. Process for the preparation of a compound of the general formula I as defined in claim 1 wherein Z represents —CO—CO—, —CO—S—, or —CO—CHOH—, which comprises warming a urea of formula III.

$$R_1—NH—CO—NH—R_2 \qquad III$$

with, respectively, oxalyl chloride, (chlorothio)formylchloride, or glyoxylic acid in the presence of an inert solvent.

8. Process as claimed in claim 7 wherein, when a chloride is used, the reaction is carried out in the presence of a tertiary nitrogen base as hydrogen chloride acceptor.

9. Process for the preparation of a compound of the general formula I as defined in claim 1 wherein Z represents $—CH_2OCH_2—$ which comprises reacting a urea formula III as defined in claim 7 with paraformaldehyde in an inert solvent and in the presence of an acid as catalyst.

10. Process for the preparation of a compound of the general formula I as defined in claim 1 wherein Z represents $—CH_2N(R_3)—CH_2—$ which comprises reacting a urea of formula III as defined in claim 7 with aqueous formaldehyde and reacting the resulting mixture with an amine of formula $R_3NH_2$.

11. A compound as claimed in claim 1, whenever prepared by a process as claimed in any one of claims 7—10.

12. Herbicidal composition, which comprises a compound as claimed in any one of claims 1—6 and 11, together with a carrier.

13. A composition as claimed in claim 12, which comprises at least two carriers, at least one of which is a surface-active agent.

14. Method of combating undesired plant growth at locus, which comprises treating the locus with a

compound as claimed in any one of claims 1 to 6 and 11, or a composition as claimed in either of claims 12 or 13.

15. The use of a compound as claimed in any one of claims 1 to 6 and 11 as a herbicide.

**Claims for the Contracting State: AT**

1. A herbicidal composition comprising a carrier and an active ingredient of the formula:—

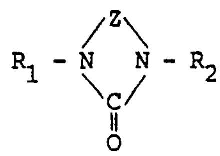

(I)

wherein

Z is a bivalent group of the formula —CO—CO—, —CO—S—, —CO—CHOH—, or $—CH_2—X—CH_2$;

X is an oxygen atom or the group $N—R_3$ wherein $R_3$ is an alkyl group of from 1 to 3 carbon atoms, or a cyclopropyl or methylcyclopropyl group;

one of $R_1$ and $R_2$ is an oxazolyl group of formula IIA

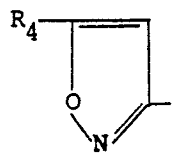

IIA

or, when Z is —CO—CO—, or —CO—CHOH—, may alternatively be the isomeric formula IIB

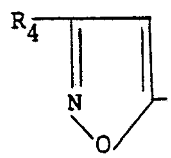

IIB

and the other is an alkyl, haloalkyl or alkoxy group of 1 to 3 carbon atoms, a cycloalkyl group of 3 to 6 carbon atoms or an alkenyl group of 3 to 5 carbon atoms; and

$R_4$ is a group containing 3 to 5 carbon atoms which is an alkyl, alkenyl, alkynyl, alkoxyalkyl, alkylthioalkyl, alkylsulphinylalkyl, alkylsulphonylalkyl, cycloalkyl, alkylcycloalkyl or haloalkyl group;

provided that when Z is —CO—CHOH then $R_2$ is not haloalkyl and $R_4$ is not alkoxyalkyl; and when Z is $—CH_2—X—CH_2—$ then $R_4$ is not haloalkyl.

2. A composition as claimed in claim 1 wherein one of $R_1$ and $R_2$ represents an alkyl or alkoxy group.

3. A composition as claimed in claim 1 or 2 wherein $R_4$ represents an alkyl, alkynyl, alkoxyalkyl or alkylcycloalkyl group.

4. A composition as claimed in claim 2 and 3 wherein one of $R_1$ and $R_2$ represents methyl or methoxy and $R_4$ represents a butyl, ethynylpropyl, methoxybutyl or methylcyclopropyl group.

5. A composition as claimed in claim 4 wherein $R_4$ is tertiary butyl.

6. A composition as claimed in any one of the preceding claims wherein the oxazolyl substituent has the isomeric form given in formula IIA.

7. A composition as claimed in any preceding claim, which comprises at least two carriers, at least one of which is a surface-active agent.

8. Process for the preparation of a compound of the general formula I as defined in claim 1 wherein Z represents —CO—CO—, —CO—S—, or —CO—CHOH—, which comprises warming a urea of formula III.

$$R_1—NH—CO—NH—R_2 \qquad III$$

with, respectively, oxalyl chloride, (chlorothio)formylchloride, or glyoxylic acid in the presence of an inert solvent.

9. Process as claimed in claim 8 wherein, when a chloride is used, the reaction is carried out in the presence of a tertiary nitrogen base as hydrogen chloride acceptor.

10. Process for the preparation of a compound of the general formula I as defined in claim 1 wherein Z represents $—CH_2OCH_2—$ which comprises reacting a urea of formula III as defined in claim 7 with paraformaldehyde in an inert solvent and in the presence of an acid as catalyst.

11. Process for the preparation of a compound of the general formula I as defined in claim 1 wherein Z represents $—CH_2N(R_3)—CH_2—$ which comprises reacting a urea formula III as defined in claim 7 with aqueous formaldehyde and reacting the resulting mixture with an amine of formula $R_3NH_2$.

12. Method of combating undesired plant growth at locus, which comprises treating the locus with a compound as defined in any of claims 1 to 6.

13. The use of a compound as defined in any one of claims 1 to 6, as a herbicide.

**Patentansprüche für die Vertragsstaaten: BE CH LI DE FR GB IT LU NL SE**

1. Eine Verbindung der Formel:

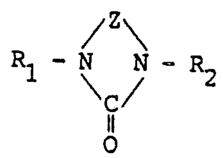

(I)

worin

Z eine zweiwertige Gruppe der Formel —CO—CO—, —CO—S—, —CO—CHOH— oder $—CH_2—X—CH_2—$ darstellt;

X ein Sauerstoffatom oder die Gruppe $N—R_3$ darstellt, worin $R_3$ eine Alkylgruppe mit 1 bis 3 Kohlenstoffatomen bedeutet, oder eine Cyclopropyl- oder Methylcyclopropylgruppe darstellt;

einer der Reste $R_1$ und $R_2$ eine Oxazolylgruppe der Formel IIA

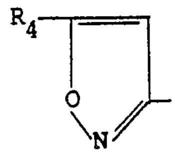

IIA

oder, wenn Z für —CO—CO— oder —CO—CHOH— steht, alternativ der isomeren Formel IIB

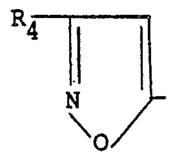

IIB

bedeutet und der andere Rest eine Alkyl-, Halogenalkyl- oder Alkoxygruppe mit 1 bis 3 Kohlenstoffatomen, eine Cycloalkylgruppe mit 3 bis 6 Kohlenstoffatomen oder eine Alkenylgruppe mit 3 bis 5 Kohlenstoffatomen darstellt; und

$R_4$ eine 3 bis 5 Kohlenstoffatome enthaltende Gruppe bedeutet, die eine Alkyl-, Alkenyl, Alkinyl-, Alkoxylalkyl-, Alkylthioalkyl-, Alkylsulfinylalkyl-, Alkylsulfonyalkyl-, Cycloalkyl-, Alkylcycloalkyl- oder Halogenalkylgruppe ist;

mit der Maßgabe, daß dann, wenn Z für —CO—CHOH steht, $R_2$ nicht Halogenalkyl ist und $R_4$ nicht Alkoxyalkyl ist; und dann,

wenn Z für $—CH_2—X—CH_2—$ steht, $R_4$ nicht Halogenalkyl bedeutet.

2. Verbindung nach Anspruch 1, worin einer der Reste $R_1$ und $R_2$ eine Alkyl- oder Alkoxygruppe bedeutet.

3. Verbindung nach Anspruch 1 oder 2, worin $R_4$ eine Alkyl-, Alkinyl-, Alkoxyalkyl- oder Alkylcycloalkylgruppe bedeutet.

4. Verbindung nach Anspruch 2 und 3, worin einer der Reste $R_1$ und $R_2$ Methyl oder Methoxy bedeutet und $R_4$ eine Butyl-, Ethinylpropyl-, Methoxybutyl- oder Methylcyclopropylgruppe darstellt.

5. Verbindung nach Anspruch 4, worin $R_4$ für tertiäres Butyl steht.

6. Verbindung nach einem der vorstehenden Ansprüche, worin der Oxazolylsubstituent die in Formel IIA angegebene Isomerenform aufweist.

7. Verfahren zur Herstellung einer Verbindung der allgemeinen Formel I, wie in Anspruch 1 definiert, worin Z für —CO—CO—, —CO—S— oder —CO—CHOH— steht, welches ein Erwärmen eines Harnstoffes der Formel III

$$R_1—NH—CO—NH—R_2 \qquad (III)$$

mit Oxalylchlorid, (Chlorthio)formylchlorid bzw. Glyoxylsäure in Gegenwart eines inerten Lösungsmittels umfasst.

8. Verfahren nach Anspruch 7, worin bei Verwendung eines Chlorids die Umsetzung in Gegenwart einer tertiären Stickstoffbase als Chlorwasserstoffakzeptor ausgeführt wird.

9. Verfahren zur Herstellung einer Verbindung der allgemeinen Formel I, wie in Anspruch 1 definiert, worin Z für $—CH_2OCH_2—$ steht, welches ein Umsetzen eines Harnstoffes der Formel III, wie in Anspruch 7 definiert, mit Paraformaldehyd in einem inerten Lösungsmittel und in Gegenwart einer Säure als Katalysator umfasst.

10. Verfahren zur Herstellung einer Verbindung der allgemeinen Formel I, wie in Anspruch 1 definiert, worin Z für $—CH_2N(R_3)—CH_2—$, steht, welches ein Umsetzen eines Harnstoffes der Formel III, wie in

Anspruch 7 definiert, mit wäßrigem Formaldehyd une ein Umsetzen des erhaltenen Gemisches mit einem Amin der Formel $R_3NH_2$ umfasst.

11. Verbindung gemäß Definition in Anspruch 1, wann immer hergestellt nach einem Verfahren, wie in einem der Ansprüche 7—10 beansprucht.

12. Herbizide Zusammensetzung, welche eine Verbindung, wie in einem der Ansprüche 1—6 und 11 beansprucht, zusammen mit einem Träger umfasst.

13. Zusammensetzung nach Anspruch 12, welche wenigstens zwei Träger umfasst, von denen wenigstens einer ein oberflächenaktives Mittel ist.

14. Verfahren zur Bekämpfung von unerwünschtem Pflanzenwuchs an einem Ort, welches ein Behandeln des Ortes mit einer Verbindung, wie in einem der Ansprüche 1 bis 6 und 11 beansprucht, oder mit einer Zusammensetzung, wie in Anspruch 12 oder 13 beansprucht, umfasst.

15. Verwendung einer Verbindung, wie in einem der Ansprüche 1 bis 6 und 11 beansprucht, als Herbizid.

**Patentansprüche fün den Vertragsstaat: AT**

1. Herbizide Zusammensetzung, umfassend einen Träger und einen wirksamen Bestandteil der Formel:

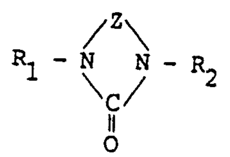

(I)

worin

Z eine zweiwertige Gruppe der Formel —CO—CO—, —CO—S—, —CO—CHOH— oder $—CH_2—X—CH_2—$ darstellt;

X ein Sauerstoffatom oder die Gruppe $N—R_3$ darstellt, worin $R_3$ eine Alkylgruppe mit 1 bis 3 Kohlenstoffatomen bedeutet, oder eine Cyclopropyl- oder Methylcyclopropylgruppe darstellt;

einer der Reste $R_1$ und $R_2$ eine Oxazolylgruppe der Formel IIA

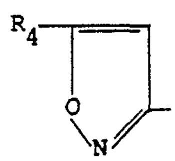

IIA

oder, wenn Z für —CO—CO— oder —CO—CHOH— steht, alternativ der isomeren Formel IIB

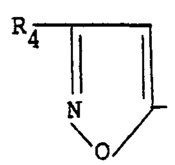

IIB

bedeutet und der andere Rest eine Alkyl-, Halogenalkyl- oder Alkoxygruppe mit 1 bis 3 Kohlenstoffatomen, eine Cycloalkylgruppe mit 3 bis 6 Kohlenstoffatomen oder eine Alkenylgruppe mit 3 bis 5 Kohlenstoffatomen darstellt; und

$R_4$ eine 3 bis 5 Kohlenstoffatome enthaltende Gruppe bedeutet, die eine Alkyl-, Alkenyl, Alkinyl-, Alkoxylalkyl-, Alkylthioalkyl-, Alkylsulfinylalkyl-, Alkylsulfonyalkyl-, Cycloalkyl-, Alkylcycloalkyl- oder Halogenalkylgruppe ist;

mit der Maßgabe, daß dann, wenn Z für —CO—CHOH steht, $R_2$ nicht Halogenalkyl ist und $R_4$ nicht Alkoxyalkyl ist; und dann,

wenn Z für $—CH_2—X—CH_2—$ steht, $R_4$ nicht Halogenalkyl bedeutet.

2. Zusammensetzung nach Anspruch 1, worin einer der Reste $R_1$ und $R_2$ eine Alkyl- oder Alkoxygruppe bedeutet.

3. Zusammensetzung nach Anspruch 1 oder 2, worin $R_4$ eine Alkyl-, Alkinyl-, Alkoxyalkyl- oder Alkylcycloalkylgruppe bedeutet.

4. Zusammensetzung nach Anspruch 2 und 3, worin einer der Reste $R_1$ und $R_2$ Methyl oder Methoxy bedeutet und $R_4$ eine Butyl-, Ethinylpropyl-, Methoxybutyl- oder Ethylcyclopropylgruppe darstellt.

5. Zusammensetzung nach Anspruch 4, worin $R_4$ für tertiäres Butyl steht.

6. Zusammensetzung nach einem der vorstehenden Ansprüche, worin der Oxazolylsubstituent die in Formel IIA angegebene Isomerenform aufweist.

7. Zusammensetzung nach einem der vorstehenden Ansprüche, welche wenigstens zwei Träger umfasst, von denen wenigstens einer ein oberflächenaktives Mittel ist.

8. Verfahren zur Herstellung einer Verbindung der allgemeinen Formel I, wie in Anspruch 1 definiert, worin Z für —CO—CO—, —CO—S— oder —CO—CHOH— steht, welches ein Erwärmen eines Harnstoffes der Formel III

$$R_1—NH—CO—NH—R_2 \qquad (III)$$

mit Oxalylchlorid, (Chlorthio)formylchlorid bzw. Glyoxylsäure in Gegenwart eines inerten Lösungsmittels umfasst.

9. Verfahren nach Anspruch 8, worin bei Verwendung eines Chlorids die Umsetzung in Gegenwart einer tertiären Stickstoffbase als Chlorwasserstoffakzeptor ausgeführt wird.

10. Verfahren zur Herstellung einer Verbindung der allgemeinen Formel I, wie in Anspruch 1 definiert, worin Z für $—CH_2OCH_2—$ steht, welches ein Umsetzen eines Harnstoffes der Formel III, wie in Anspruch 8 definiert, mit Paraformaldehyd in einem inerten Lösungsmittel und in Gegenwart einer Säure als Katalysator umfasst.

11. Verfahren zur Herstellung einer Verbindung der allgemeinen Formel I, wie in Anspruch 1 definiert, worin Z für $—CH_2N(R_3)—CH_2—$ steht, welches ein Umsetzen eines Harnstoffes der Formel III, wie in Anspruch 8 definiert, mit wäßrigem Formaldehyd und ein Umsetzen des erhaltenen Gemisches mit einem Amin der Formel $R_3NH_2$ umfasst.

12. Verfahren zur Bekämpfung von unerwünschtem Pflanzenwuchs an einem Ort, welches ein Behandeln des Ortes mit einer Verbindung, wie in einem der Ansprüche 1—6 definiert, umfasst.

13. Verwendung einer Verbindung, wie in einem der Ansprüche 1—6 definiert, als Herbizid.

**Revendications pour les Etats contractants: BE CH LI DE FR GB IT LU NL SE**

1. Un composé de la formule:

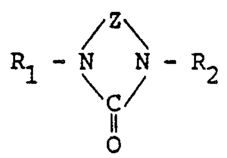

(I)

où

Z est un groupe bivalent de la formule —CO—CO—, —CO—S—, —CO—CHOH ou $—CH_2—X—CH_2$;

X est un atome d'oxygène ou le groupe $N—R_3$ où $R_3$ est un groupe alkyle de 1 à 3 atomes de carbone ou un groupe cyclopropyle ou méthylcyclopropyle;

un de $R_1$ et $R_2$ est un groupe oxazolyle de formule IIA

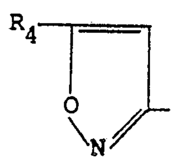

IIA

ou, quand Z est —CO—CO— ou —CO—CHOH—, peut aussi être de la formule isomère IIB

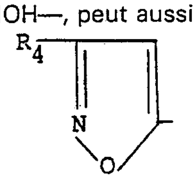

IIB

et l'autre est un groupe alkyle, haloalkyle ou alcoxy de 1 à 3 atomes de carbone, un groupe cycloalkyle de 3 à 6 atomes de carbone ou un groupe alcényle de 3 à 5 atomes de carbone; et

$R_4$ est un groupe contenant 3 à 5 atomes de carbone qui est un groupe alkyle, alcényle, alcynyle, alcoxyalkyle, alkylthioalkyle, alkylsulfinylalkyle, alkylsulfonylalkyle, cycloalkyle, alkylcycloalkyle ou haloalkyle;

avec les conditions que quand Z est —CO—CHOH, alors $R_2$ n'est pas un groupe haloalkyle et $R_4$ n'est pas un groupe alcoxyalkyle;

et quand Z est $—CH_2—X—CH_2—$, alors $R_4$ n'est pas un groupe haloalkyle.

2. Un composé selon la revendication 1, dans lequel un de $R_1$ et $R_2$ représente un groupe alkyle ou alcoxy.

3. Un composé selon la revendication 1 ou 2, dans lequel $R_4$ représente un groupe alkyle, alcynyle, alcoxyalkyle ou alkylcycloalkyle.

4. Un composé selon la revendication 2 ou 3, dans lequel un de $R_1$ et $R_2$ représente un groupe méthyle ou méthoxy et $R_4$ représente un groupe butyle, éthynylpropyle, méthoxybutyle ou méthylcyclopropyle.

5. Un composé selon la revendication 4, dans lequel $R_4$ est un groupe tertiobutyle.

6. Un composé selon l'une quelconque des revendications précédentes, dans lequel le substituant oxazolyle a la forme isomère indiquée dans la formule II.

7. Procédé pour la préparation d'un composé de la formule générale I tel que défini dans la revendication 1 dans lequel Z représente —CO—CO—, —CO—S— ou —CO—CHOH—, qui comprend le chauffage d'une urée de formule III:

$$R_1—NH—CO—NH—R_2 \qquad III$$

avec, respectivement, du chlorure d'oxalyle, du chlorure de (chlorothio)formyle ou de l'acide glyoxylique en présence d'un solvant inerte.

8. Procédé selon la revendication 7, dans lequel, quand on utilise un chlorure, la réaction est conduite en présence d'une base azotée tertiaire comme accepteur de chlorure d'hydrogène.

9. Procédé pour la préparation d'un composé de la formule générale I tel que défini dans la revendication 1 dans lequel Z représente —$CH_2OCH_2$—, qui comprend la réaction d'une urée de formule III telle que définie dans la revendication 7 avec du paraformaldéhyde dans un solvant inerte et en présence d'un acide comme catalyseur.

10. Procédé pour la préparation d'un composé de la formule générale I tel que défini dans la revendication 1 dans lequel Z représente —$CH_2N(R_3)$—$CH_2$, qui comprend la réaction d'une urée de formule III telle que définie dans la revendication 7 avec du formaldéhyde aqueux et la réaction du mélange résultant avec une amine de formule $R_3NH_2$.

11. Un composé tel que défini dans la revendication 1, chaque fois qu'il est préparé par un procédé selon l'une quelconque des revendications 7—10.

12. Composition à activité herbicide, qui comprend un composé tel que revendiqué dans l'une quelconque des revendications 1—6 et 11, en même temps qu'un véhicule.

13. Une composition selon la revendication 12, qui comprend au moins deux véhicules, dont au moins un est un agent tensio-actif.

14. Méthode de lutte contre la croissance de plantes indésirables en un lieu, qui comprend la traitement du lieu par un composé selon l'une quelconque des revendications 1 à 6 et 11, ou par une composition selon l'une des revendications 12 ou 13.

15. L'utilisation d'un composé selon l'une quelconque des revendications 1 à 6 et 11 comme herbicide.

**Revendications pour l'Etat contractant: AT**

1. Une composition à activité herbicide comprenant un véhicule et un ingrédient actif de la formule:

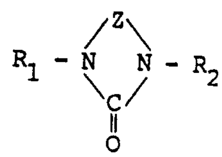

(I)

où

Z est un groupe bivalent de la formule —CO—CO—, —CO—S—, —CO—CHOH— ou —$CH_2$—X—$CH_2$;

X est un atome d'oxygène ou le groupe N—$R_3$ où $R_3$ est un groupe alkyle de 1 à 3 atomes de carbone ou un groupe cyclopropyle ou méthylcyclopropyle;

un de $R_1$ et $R_2$ est un groupe oxazolyle de formule IIA

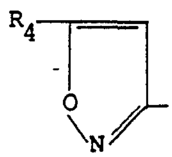

IIA

ou, quand Z est —CO—CO— ou —CO—CHOH—, peut aussi être de la formule isomere IIB

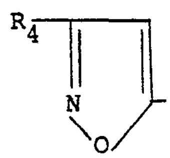

IIB

et l'autre est un groupe alkyle, haloalkyle ou alcoxy de 1 à 3 atomes de carbone, un groupe cycloalkyle de 3 à 6 atomes de carbone ou un groupe alcényle de 3 à 5 atomes de carbone; et

$R_4$ est un groupe contenant 3 à 5 atomes de carbone qui est un groupe alkyle, alcényle, alcynyle, alcoxyalkyle, alkylthioalkyle, alkylsulfinylalkyle, alkylsulfonylalkyle, cycloalkyle, alkylcycloalkyle ou haloalkyle;

avec les conditions que quand Z est —CO—CHOH, alors $R_2$ n'est pas un groupe haloalkyle et $R_4$ n'est pas un groupe alcoxyalkyle;

et quand Z est —$CH_2$—X—$CH_2$—, alors $R_4$ n'est pas un groupe haloalkyle.

2. Une composition selon la revendication 1, dans laquelle un de $R_1$ et $R_2$ représente un groupe alkyle ou alcoxy.

3. Une composition selon la revendication 1 ou 2, dans laquelle $R_4$ représente un groupe alkyle, alcynyle, alcoxyalkyle ou alkylcycloalkyle.

4. Une composition selon les revendications 2 et 3, dans laquelle un de $R_1$ et $R_2$ représente un groupe méthyle ou méthoxy et $R_4$ représente un groupe butyle, éthynylpropyle, méthoxybutyle ou méthylcyclopropyle.

5. Une composition selon la revendication 4, dans laquelle $R_4$ est un groupe tertiobutyle.

6. Une composition selon l'une quelconque des revendications précédentes, dans laquelle le substituant oxazolyle a la forme isomère indiquée dans la formule IIA.

7. Une composition selon l'une quelconque des revendications précédentes, qui comprend au moins deux véhicules, dont l'un au moins est un agent tensio-actif.

8. Procédé pour la préparation d'un composé de la formule générale I tel que défini dans la revendication 1 dans lequel Z représente —CO—CO—, —CO—S— ou —CO—CHOH—, qui comprend le chauffage d'une urée de formule III:

$$R_1—NH—CO—NH—R_2 \qquad III$$

avec, respectivement, du chlorure d'oxalyle, du chlorure de (chlorothio)formyle ou de l'acide glyoxylique en présence d'un solvant inerte.

9. Procédé selon la revendication 8, dans lequel, quand on utilise un chlorure, la réaction est conduite en présence d'une base azotée tertiaire comme accepteur de chlorure d'hydrogène.

10. Procédé pour la préparation d'un composé de la formule générale I tel que défini dans la revendication 1 dans lequel Z représente —$CH_2OCH_2$—, qui comprend la réaction d'une urée de formule III telle que définie dans la revendication 7 avec du paraformaldéhyde dans un solvant inerte et en présence d'un acide comme catalyseur.

11. Procédé pour la préparation d'un composé de la formule générale I tel que défini dans la revendication 1 dans lequel Z représente —$CH_2N(R_3)$—$CH_2$—, qui comprend la réaction d'une urée de formule III telle que définie dans la revendication 7 avec du formaldéhyde aqueux et la réaction du mélange résultant avec une amine de formule $R_3NH_2$.

12. Méthode de lutte contre la croissance de plantes indésirables en un lieu, qui comprend la traitement du lieu par un composé tel que défini dans l'une quelconque des revendications 1 à 6.

13. L'utilisation d'un composé tel que défini dans l'une quelconque des revendications 1 à 6 comme herbicide.